# EUROPÄISCHE PATENTANMELDUNG

(11) **EP 4 215 216 A1**
(43) Veröffentlichungstag der Anmeldung: **26.07.2023**
(21) Anmeldenummer: 23152583.3
(22) Anmeldetag: 20.01.2023
(51) Int. Cl.: A61L 2/18, C11D 17/04, A47L 13/17

(54) **DESINFEKTIONSSYSTEM MIT EINEM TUCH AUS EINEM POLYPROPYLEN AUFWEISENDEN THERMOBOND-VLIESSTOFF**

(30) Priorität: 21.01.2022 DE 102022101454
(71) Anmelder: Schülke & Mayr GmbH, 22851 Norderstedt (DE)
(72) Erfinder: Jordan, Heike, 22851 Norderstedt (DE); Teutenberg, Lars, 22851 Norderstedt (DE)
(74) Vertreter: LKGLOBAL Lorenz & Kopf Patentanwalt Attorney at Law PartG mbB

(57) **Zusammenfassung**

Ein Desinfektionssystem zur Flächendesinfektion weist ein Tuch mit einem Trägermaterial aus einem thermisch verfestigten Vliesstoff, oder Thermobond-Vliesstoff, der Polypropylen aufweist, und eine Desinfektionslösung auf, die Peressigsäure aufweist, wobei das Tuch mit der Desinfektionslösung tränkbar ist.

## Beschreibung

### Technisches Gebiet

Die vorliegende Erfindung betrifft ein Desinfektionssystem zur Desinfektion von Oberflächen aufweisend ein Tuch mit einem Trägermaterial aus einem thermisch verfestigten Vliesstoff, oder Thermobond-Vliesstoff, der Polypropylen aufweist sowie die Verwendung eines Tuchs mit einem Trägermaterial aus einem thermisch verfestigten Vliesstoff oder Thermobond-Vliesstoff, der Polypropylen aufweist.

### Technischer Hintergrund

Zur Schnelldesinfektion von Medizinprodukten und wischbaren Oberflächen aller Art mit erhöhtem Infektionsrisiko, wie beispielsweise Sanitäroberflächen oder Oberflächen in Krankenhäusern, Arztpraxen, etc., unter Berücksichtigung des Erfordernisses kurzer Einwirkzeiten ist bekannt, mit einer Wirkstofflösung getränkte Desinfektionstücher einzusetzen. Diese sind oftmals gebrauchsfertig getränkt und in einem Aufbewahrungsbehältnis gelagert, welches eine fluiddichte Aufbewahrung und eine Einzelentnahme von Tüchern erlaubt. Es sind weiterhin Systeme bekannt, bei denen trockene Tücher in einem Aufbewahrungsbehältnis bereitgestellt werden und vor einer Benutzungsaufnahme in dem Aufbewahrungsbehältnis mit einer separat bereitgestellten Desinfektionslösung getränkt werden.

Ebenfalls bekannt ist, dass zur Desinfektion auf Persäuren basierende Desinfektionslösungen, insbesondere auf Peressigsäure (PAA) basierende Desinfektionslösungen verwendet werden können. Die Persäuren weisen allerdings als solche in der Regel schon eine gewisse Instabilität auf und neigen mit der Zeit zur Zersetzung, insbesondere wenn sie Licht und höheren Temperaturen ausgesetzt sind. Es wurde zudem beobachtet, dass es nach der Tränkung von zur Oberflächendesinfektion üblichem Vliesstoff-Material mit einer Persäure-Lösung, beispielsweise mit einer PAA-Lösung zusätzlich zu einem vor allem anfänglich kontinuierlichen Abbau der Peressigsäure auch bei Raumtemperatur und ohne Lichtaussetzung kommen kann, sodass dadurch die Wirksamkeit und Haltbarkeit von mit Persäure-Lösung, beispielsweise mit PAA-Lösung, getränkten, gebrauchsfertigen Tüchern beschränkt ist.

### Beschreibung

Es kann als Aufgabe betrachtet werden, die Stabilität von gebrauchsfertigen Tüchern zu verbessern, die mit einer Persäure-Lösung, beispielsweise einer PAA-Lösung, getränkt sind, um die Wirksamkeit und/oder die Haltbarkeit zu erhöhen.

Diese Aufgabe wird gelöst durch den Gegenstand des unabhängigen Anspruchs 1. Weitere Ausführungsformen ergeben sich aus den abhängigen Ansprüchen sowie aus der folgenden Beschreibung.

Es wird ein Desinfektionssystem zur Flächendesinfektion vorgeschlagen, aufweisend ein Tuch mit einem Trägermaterial aus einem Thermobond-Vliesstoff, der Polypropylen aufweist, und eine Desinfektionslösung, die eine Persäure, bevorzugt Peressigsäure, umfasst, wobei das Tuch mit der Desinfektionslösung tränkbar ist.

Das Desinfektionssystem umfasst folglich zumindest ein tränkbares Tuch oder mehrere tränkbare Tücher aus einem vorteilhaften Trägermaterial und eine entsprechende Desinfektionslösung, mit der das zumindest eine Tuch oder die Tücher tränkbar sind.

Das zumindest eine Tuch oder die mehreren Tücher umfassen ein Trägermaterial aus einem thermisch verfestigten Vliesstoff, der nachfolgend als Thermobond-Vliesstoff benannt ist. Zur Herstellung eines Thermobond-Vliesstoffs werden Fasern zu einem Flor gelegt und ausgerichtet. Sie werden anschließend kalandriert und unter Einwirkung von Wärme und Druck verfestigt.

Der besondere Vorteil der Verwendung eines Thermobond-Vliesstoffes liegt in der deutlichen Verbesserung der Stabilität der Desinfektionslösung im gebundenen Zustand am Vliesstoff, der Haltbarkeit eines vorgetränkten Tuchs in einem geschlossenen Aufbewahrungsbehältnis und der Wirksamkeit eines getränkten Tuchs zur Oberflächendesinfektion. Dabei spielt der Thermobond-Vliesstoff eine entscheidende Rolle, denn es wurde überraschend festgestellt, dass der Wirkstoffabfall eines daraus hergestellten und anschließend getränkten Tuchs im Vergleich zu getränkten Tüchern, die ein Trägermaterial aus einem Meltblown-Verfahren aufweisen, deutlich reduziert wird. Dies betrifft beispielsweise und insbesondere die Wirkungsbereiche der Sporizide gemäß EN14476 und der Mycobacterizide gemäß EN 14348. Ein nach dem erfindungsgemäßen Prinzip getränktes Tuch erlaubt aufgrund des geringeren Wirkstoffabfalls, d.h. eines effektiv höheren eine Persäuregehalts, bevorzugt Peressigsäuregehalts, eine schnellere Wirksamkeit, wodurch beispielsweise die benötigte Einwirkzeit der durch das getränkte Tuch auf eine zu desinfizierende Oberfläche aufgebrachten Persäure, bevorzugt Peressigsäure, enthaltenden Lösung von 15 Minuten auf 5 Minuten reduziert werden kann.

Zudem ist eine verlängerte Haltbarkeit gebrauchsfertig getränkter Tücher in dem geschlossenen Aufbewahrungsbehältnis möglich. Die Verwendung eines Thermobond-Vliesstoffs, der für unterschiedlichste technische Anwendungen im industriellen Maßstab verfügbar ist, kann zu deutlich verringerten Materialkosten führen, welche je nach entsprechender Menge bis zu 50% im Vergleich zu herkömmlichen Trägermaterialien geringer sein könnten. Des Weiteren lässt sich ein Thermobond-Vliesstoff mit einer gleich bleibenden, stabilen Qualität stellen, sodass eine Skalierung in der Herstellung eines Desinfektionssystems vereinfacht wird.

Zudem weist der Thermobond-Vliesstoff eine sehr angenehme Optik und Haptik auf, sodass es für den Nutzer angenehmer zu verwenden ist. Der Thermobond-Vliesstoff nimmt weiterhin Flüssigkeit schneller auf, sodass ein Abfüllen bzw. Tränken des Trägermaterials deutlich verbessert und beschleunigt wird.

Das Abgabeverhalten ist weiterhin gegenüber einem herkömmlichen Trägermaterial auf Basis eines Meltblown-Verfahrens deutlich verbessert. Damit kann von einem getränkten Tuch des erfindungsgemäßen Systems insgesamt mehr Peressigsäure-enthaltende Lösung und damit Wirkstoff abgegeben werden als von herkömmlichen Tüchern. Dadurch sind potenziell kürzere Einwirkzeiten bis zum Erreichen einer akzeptablen Desinfektionswirkung und/oder größere Reichweiten, d.h. die maximal mit einem Tuch zu desinfizierende Fläche, möglich.

Das Trägermaterial aus dem Thermobond-Vliesstoff weist Polypropylen. Beispielsweise kann das Trägermaterial vollständig auf Polypropylen basieren. Die zum Herstellen des Vliesstoffs verwendeten Fasern bestehen somit in einer bevorzugten Ausführungsform ausschließlich aus Polypropylen. In alternativen Ausführungsformen können aber auch zusätzlich weitere Fasern in den Vliesstoff zugemischt werden, wobei der Anteil jedoch zur Erreichung der genannten Vorteile möglichst gering sein sollte, und bevorzugt möglichst 20 Gewichts-% nicht überschreitet. Bevorzugter enthält der Vliesstoff 15 Gewichts-% oder weniger weitere Fasern, noch bevorzugter 10 Gewichts-% oder weniger, wie beispielsweise 5 Gewichts-% oder weniger oder sogar 2 Gewichts-% oder weniger. Besonders bevorzugt enthält der Vliesstoff keine weitere Fasern und besteht somit vollständig aus Polypropylenfasern,

Das Trägermaterial kann in einer vorteilhaften Ausführungsform durch ein Trockenverfahren hergestellt sein. Die Fasern können als trockene Faserballen bereitgestellt werden, wobei die nicht miteinander verwobenen Fasern geöffnet werden, um sie in einem späteren Verfahrensschritt entsprechend auszurichten.

Das erfindungsgemäße Desinfektionssystem zur Desinfektion von Oberflächen weist eine Desinfektionslösung, die eine Persäure umfasst, auf. Prinzipiell kann in dem erfindungsgemäßen Desinfektionssystem jede Desinfektionslösung, die eine Persäure umfasst, verwendet werden.

Geeignete Persäuren sind organische Persäure, die Peroxidderivate einer oder mehrerer Carbonsäuren sind. Beispielsweise können die organische Persäuren C₁ bis C₉-Persäuren und insbesondere C₁ bis C₅-Persäuren umfassen. Beispiele für solche Persäuren umfassen Perameisensäure, Peressigsäure, Perbenzoesäure, Perpropionsäure, Pernonansäure und Halogen-substituierte Persäuren, wie beispielsweise Monochlorperessigsäure, Dichlorperessigsäure, Trichlorperessigsäure, Trifluorperessigsäure, meta-Chlorperoxybenzoesäure sowie Mischungen der genannten Persäuren. Bevorzugt umfasst die Persäure Peressigsäure und besonders bevorzugt ist die Persäure Peressigsäure.

Die Desinfektionslösung umfasst weiterhin in manchen Ausführungsformen Wasserstoffperoxid oder ein anderes Peroxid, das Wasserstoffperoxid freisetzen kann, wenn es in der Lösung vorhanden ist. Geeignete Wasserstoffperoxidquellen können beispielsweise Peroxide von Alkali- und Erdalkalimetallen, organische Peroxyverbindungen, pharmazeutisch verträgliche Salze davon und Mischungen davon umfassen. Peroxide von Alkali- und Erdalkalimetallen umfassen Lithiumperoxid, Kaliumperoxid, Natriumperoxid, Magnesiumperoxid, Calciumperoxid, Bariumperoxid und Mischungen davon. Organische Peroxykomplexe können Carbamidperoxid (auch bekannt als Harnstoffperoxid), Alkyl- und/oder Arylperoxide (beispielsweise tert-Butylperoxid, Diphenylperoxid usw.), Alkyl- und/oder Arylketonperoxide (beispielsweise Benzyolperoxid), Peroxyester, Diacylperoxide, und Mischungen davon umfassen. Bevorzugt umfasst die Desinfektionslösung Wasserstoffperoxid.

In einer besonders bevorzugten Ausführungsform umfasst die Desinfektionslösung Peressigsäure und Wasserstoffperoxid

Der Gehalt an Peroxiden, bevorzugt an Wasserstoffperoxid, in der Desinfektionslösung beträgt typischerweise 0,5 bis 15 Gewichts-%, bevorzugt 1 bis 10 Gewichts-%, in noch bevorzugter Ausführungsformen 2 bis 8 Gewichts-% und besonders bevorzugt 3 bis 6 Gewichts-%. Der Gehalt an Persäuren, bevorzugt an Peressigsäure, beträgt typischerweise 0,01 bis 2 Gewichts-%, bevorzugt 0,05 bis 1 Gewichts-% und besonders bevorzugt 0,1 bis etwa 0,5 Gewichts-%. Es versteht sich, dass die obigen Konzentrationen die Anfangskonzentrationen unmittelbar nach der Bildung der Lösung sind. Da sich Persäuren und Peroxide in Wasser zersetzen können, kann ihre Konzentration jedoch im Laufe der Zeit variieren. Nichtsdestotrotz besteht ein Vorteil der vorliegenden Erfindung darin, dass die auf das Tuch oder die mehreren Tücher eingebrachte Persäure, bevorzugt die Peressigsäure, hinreichend stabilisiert werden kann, sodass ihr Gehalt über einen bestimmten Zeitraum im Wesentlichen auf dem gleichen Niveau gehalten werden kann.

Die Desinfektionslösung kann weiterhin übliche Additive enthalten, wie beispielsweise ein Tensid zur Erhöhung der Benetzbarkeit des Trägermaterials, eine Carbonsäure, um ein Gleichgewicht mit der Persäure herzustellen, Metallchelatbildner, um die potentielle Zersetzung der Persäure durch Metallionen zu unterdrücken, etc. Im Allgemeinen kann jedes Tensid, jede Carbonsäure und jeder Metallchelatbildner verwendet werden, die weder mit der Persäure noch dem gegebenenfalls in der Desinfektionslösung enthaltenen Peroxid in einem solchen Ausmaß interagieren, dass die Stabilität der Desinfektionslösung signifikant beeinträchtigt wird.

Das Flächengewichts des Trägermaterials ist nicht weiter begrenzt. Das Trägermaterial kann aber beispielsweise ein Flächengewicht von 50 ± 30 g/m², vorzugsweise 40 ± 10 g/m², insbesondere 30 ± 3 g/m², wie etwa 30 g/m² besitzen. Damit kann eine günstige Materialstärke und eine hohe Aufnahmefähigkeit für die Desinfektionslösung erzielt werden. Eine Materialdichte kann durch eine beim Kalandrieren einstellbare Dicke des Vliesstoffs beeinflusst werden. Der Fachmann wird zur Anpassung des Tuchs an einen gewünschten Anwendungszweck und eine gewünschte Aufnahmemenge der Desinfektionslösung eine dafür geeignete Dicke bevorzugt durch Wischversuche auswählen.

Das Tuch kann als Wischtuch zur Flächendesinfektion mit einer ersten Kantenlänge in einem Bereich von 10 cm bis 30 cm, bevorzugt 14 cm bis 25 cm, und noch bevorzugter 16 cm bis 20 cm, wie beispielsweise 18cm und einer zweiten Kantenlänge in einem Bereich von 10 cm bis 0 cm, bevorzugt 15 cm bis 30 cm, noch bevorzugter 18 cm bis 25 cm, wie beispielsweise 20 cm ausgeführt sein.. Selbstverständlich können die Feuchttücher auch andere Abmessungen aufweisen, falls gewünscht oder erforderlich.

Das Tuch kann weiterhin auch als Bestandteil eines Bodentuch zur Bodendesinfektion mit einer ersten Kantenlänge in einem Bereich von 5 cm bis 30 cm, bevorzugt 8 cm bis 20 cm, noch bevorzugter 10 cm bis 15 cm, wie beispielsweise 120 cm und einer zweiten Kantenlänge in einem Bereich von 20 cm bis 80 cm, bevorzugt 25 cm bis 60 cm, noch bevorzugter 30 cm bis 50 cm, wie beispielsweise 40 cm ausgeführt sein.

Das Desinfektionssystem kann ferner einen verschließbaren Behälter zur Aufnahme mehrerer Tücher aufweisen, der dazu ausgebildet ist, Tücher einzeln aus dem Behälter zu entnehmen. Hierzu sind unterschiedliche Varianten bekannt. Größere Aufnahmebehältnisse mit starren Wandungen zum Aufbewahren trockener Tücher sind bekannt. Diese werden vor Aufnahme des Gebrauchs mit einer in das Aufnahmebehältnis einzufüllenden Desinfektionslösung getränkt, um sie anschließend einzeln und nacheinander dem Aufnahmebehältnis zu entnehmen. Es sind jedoch auch gebrauchsfertig getränkte Tücher denkbar, die beispielsweise mit einer Zickzack-Faltung ausgestattet und lagenweise in einer schlauchartigen Folienverpackung (auch als Softpack oder Flowpack bekannt) gestapelt sind.

Die Tücher könnten in dem Behälter folglich tränkbar oder vorgetränkt sein. Bevorzugt sind die Tücher in dem Behälter vorgetränkt.

Die Erfindung betrifft weiterhin die Verwendung eines Tuchs mit einem Trägermaterial aus einem Thermobond-Vliesstoff, der Polypropylen aufweist, wie oben beschrieben, und einer Desinfektionslösung, die Peressigsäure umfasst, ebenfalls wie oben beschrieben, zur Desinfektion von Oberflächen. In einer Ausführungsform wird dabei ein mit der Desinfektionslösung, die Peressigsäure umfasst, vorgetränktes Tuch mit einem Trägermaterial aus einem Thermobond-Vliesstoff, der Polypropylen aufweist, verwendet. Die Vortränkung der Tücher kann in einem Behälter, beispielsweise zur Aufbewahrung der ungetränkten Tücher, erfolgen oder der Behälter kann bereits vorgetränkte Tücher enthalten.

Das Trägermaterial ist bevorzugt vollständig aus Polypropylen hergestellt.

## Patentansprüche

1. Desinfektionssystem zur Desinfektion von Oberflächen, aufweisend:
ein Tuch mit einem Trägermaterial aus einem thermisch verfestigten Vliesstoff, oder Thermobond-Vliesstoff, der Polypropylen aufweist, und
eine Desinfektionslösung, die eine Persäure umfasst,
wobei das Tuch mit der Desinfektionslösung tränkbar ist.

2. Desinfektionssystem nach Anspruch 1, wobei die Persäure Peressigsäure ist.

3. Desinfektionssystem nach Anspruch 1 oder 2,
wobei das Trägermaterial vollständig auf Polypropylen basiert.

4. Desinfektionssystem nach einem der vorhergehenden Ansprüche,
wobei das Trägermaterial durch ein Trockenverfahren hergestellt ist.

5. Desinfektionssystem nach einem der vorhergehenden Ansprüche,
wobei das Trägermaterial ein Flächengewicht von 50 ± 30 g/m², vorzugsweise 40 ± 10 g/m², insbesondere 30 ± 3 g/m², wie etwa 30 g/m² besitzt.

6. Desinfektionssystem nach einem der vorhergehenden Ansprüche,
wobei das Tuch als Wischtuch mit einer ersten Kantenlänge in einem Bereich von 12 cm bis 30 cm, bevorzugt 20 cm und einer zweiten Kantenlänge in einem Bereich von 18 cm bis 40 cm, bevorzugt 20 cm ausgeführt ist.

7. Desinfektionssystem nach einem Ansprüche 1 bis 5,
wobei das Tuch als Bodentuch mit einer ersten Kantenlänge in einem Bereich von10 cm bis 30 cm, bevorzugt 120 cm, und einer zweiten Kantenlänge in einem Bereich von 30 cm bis 80 cm, bevorzugt 40 cm, ausgeführt ist.

8. Desinfektionssystem nach einem der vorhergehenden Ansprüche,
ferner aufweisend einen verschließbaren Behälter zur Aufnahme mehrerer Tücher, der dazu ausgebildet ist, Tücher einzeln aus dem Behälter zu entnehmen.

9. Desinfektionssystem nach Anspruch 8,
wobei die Tücher in dem Behälter tränkbar oder vorgetränkt sind, und bevorzugt vorgetränkt sind.

10. Verwendung eines Tuchs mit einem Trägermaterial aus einem thermisch verfestigten Vliesstoff, oder Thermobond-Vliesstoff, der Polypropylen aufweist, und einer Desinfektionslösung, die Peressigsäure umfasst, zur Desinfektion von Oberflächen.

11. Verwendung nach Anspruch 10, wobei das Trägermaterial vollständig aus Polypropylen hergestellt ist.

12. Verwendung nach Anspruch 10 oder 11, wobei das Trägermaterial eine glatte oder strukturierte Oberfläche hat.
